# EUROPÄISCHE PATENTSCHRIFT

(11) **EP 0 979 812 B1**
(45) Veröffentlichungstag und Bekanntmachung des Hinweises auf die Patenterteilung: **29.10.2003**
(21) Anmeldenummer: 99115025.1
(22) Anmeldetag: 02.08.1999
(51) Int. Cl.: C07C 45/63, C07C 29/141, C07C 33/46

(54) **Verfahren zur Herstellung von fluorierten Benzylalkoholen und -aldehyden**
Process for the preparation of fluorinated benzyl alcools and benzyl aldehydes
Procédé de préparation d'alcools et d'aldehydes benzyliques fluorés

(30) Priorität: 13.08.1998 DE 19836698
(43) Veröffentlichungstag der Anmeldung: 16.02.2000
(73) Patentinhaber: Bayer Aktiengesellschaft, 51368 Leverkusen (DE)
(72) Erfinder: Wiedemann, Jürgen, Dr., 50127 Bergheim (DE); Marhold, Albrecht, Dr., 51373 Leverkusen (DE); Dreisbach, Claus, Dr., 51065 Köln (DE)

(56) Entgegenhaltungen:
- DE-A- 3 637 156
- DE-A- 19 702 282
- J. ENGLISH ET AL.: "The synthesis of 3,5-difluoro- and 3-fluoro-5-iodo-dl-tyrosine " J.AMERICAN CHEM. SOC., Bd. 62, 1949, Seite 350-353 XP002124525

## Beschreibung

Die vorliegende Erfindung betrifft ein verbessertes Verfahren zur Herstellung von fluorierten Benzylalkoholen in hohen Ausbeuten und mit günstigen Kosten über die entsprechenden fluorierten Benzaldehyde aus den entsprechenden chlorierten Benzaldehyden.

Fluorierte Benzylalkohole sind nicht nur wichtige Vorprodukte für die Herstellung von Pharmazeutika und Pflanzenschutzmitteln (WO 95/19 980, DE-A 27 14 042), sondern weisen auch schistosomacidale Wirkung auf (US-A 3 855 291) und werden als Komponente in mikrobiziden Mitteln verwendet (DE-A-233 849, US-A 4 167 583).

Aus der EP-B 265 854 ist bekannt, daß man aus Chlorbenzaldehyden mit Alkalifluoriden in einem dipolar-aprotischen Lösungsmittel Fluorbenzaldehyde herstellen kann. Die Ausbeute beträgt dabei 68% der Theorie, was unbefriedigend ist. Außerdem beträgt dabei das Gewichtsverhältnis von Edukt zu Lösungsmittel 1:5,7, woraus eine schlechte Raum-Zeit-Ausbeute resultiert.

Die EP-B 523 668 beschreibt die Herstellung von Difluorbenzaldehyden aus Dichlorbenzaldehyden durch Umsetzung mit Alkalimetallfluoriden in einem dipolar-aprotischen Lösungsmittel in Gegenwart von Ethylenglykoldialkylethern als Katalysator. Der Katalysator wird in Mengen von 10 bis 50 g pro Mol Dichlorbenzaldehyd eingesetzt. Die Ausbeute an Difluorbenzaldehyden liegt schon beim ungereinigten Produkt immer unter 75 %. Das Lösungsmittel wird in einer Menge von 400 g pro Mol Dichlorbenzaldehyd verwendet, was 2,5 Mol Dichlorbenzaldehyd pro kg Lösungsmittel entspricht. Nachteilig sind die hohen Katalysator- und Lösungsmittelmengen mit denen nur eine nicht befriedigende Ausbeute erzielt wird.

Gemäß der EP-B 289 942 werden Fluorbenzaldehyde hergestellt, indem man Chlorbenzaldehyde in Gegenwart von quartären Phosphoniumsalzen und/oder quartären Ammoniumsalzen und gegebenenfalls weiteren Katalysatoren mit Metallfluoriden in Substanz oder in aromatischen Kohlenwasserstoffen umsetzt. Die Katalysatoren werden in Mengen von 5 bis 50 Mol-%, bezogen auf den Chlorbenzaldehyd, eingesetzt. Die Ausbeuten liegen im Bereich von 41 bis 75 %, erreichen aber nur dann Werte über 55 %, wenn ein Gemisch aus zwei unterschiedlichen Katalysatortypen eingesetzt wird. Selbst dann sind die Ausbeuten noch nicht befriedigend.

Die bisher beschriebenen Verfahren des Standes der Technik zur Herstellung von fluorierten Benzaldehyden erfordern alle hohe Temperaturen und liefern Produkte, die nur sehr schwer zu entfernende Nebenkomponenten enthalten. Beides ist nachteilig; hohe Temperaturen, weil sie viel Energie erfordern, und nur schwierig zu entfernende Nebenkomponenten, weil diese bei Zwischenprodukten für Wirkstoffe nicht toleriert werden können. Senkt man die Temperatur ab, z.B. beim Verfahren entsprechend der DE-A 36 37 156, so sinkt die Ausbeute auch bei der Einhaltung langer Reaktionszeiten drastisch ab (siehe Vergleichsbeispiel).

Es ist ferner bekannt, fluorierte Benzylalkohole aus den entsprechenden Benzoesäuren durch Reduktion mit Lithiumaluminiumhydrid oder aus den entsprechenden Benzoesäurechloriden durch Reduktion mit Alkaliboranaten zu gewinnen. Als Ausgangsmaterialien dienen dabei fluorsubstituierte Säurechloride, bei denen das Fluor über eine Halogen-Austausch-Reaktion eingeführt wurde oder Aminobenzoesäuren, welche durch Diazotierung in Fluorwasserstoff oder nach der Methode von Balz-Schiemann in Fluor-substituierte Derivate überführt wurden (siehe Houben-Weyl, Methoden der organischen Chemie, 4. Auflage, Band 5/3, Seiten 215 und 227). Alle diese Verfahren verlangen bei der Reduktion zum Benzylalkohol die Verwendung von Metallhydridreagenzien und sind daher technisch nur schwierig durchführbar und außerdem teuer.

Eine weitere Quelle von fluorierten Benzylalkoholen sind die entsprechenden Benzylchloride oder -bromide, welche durch Seitenkettenhalogenierung der entsprechenden fluorierten Toluole erhalten werden können. Diese Toluole sind jedoch oft nur schwer zugänglich oder nur aus den hier interessierenden Produkten herstellbar.

In der DE-A 2 333 849 ist beschrieben, daß 2,4-Difluorbenzaldehyd durch Hydrierung an Raney-Nickel mit 68,6 %iger Ausbeute in den 2,4-Difluorbenzylalkohol überführt werden kann. Nachteilig bei diesem Verfahren ist neben der großen Katalysatormenge (31 g pro Mol) und der großen Lösungsmittelmenge (1100 ml pro Mol) die Ausbeute von 68,6 %. Diese Faktoren bedingen eine ungenügende Raum-Zeit-Ausbeute. Der eingesetzte Aldehyd wurde dabei durch Seitenkettenchlorierung mit anschließender Hydrolyse aus dem schwierig zugänglichen 2,4-Difluortoluol gewonnen.

Zusammenfassend ist hinsichtlich des Standes der Technik festzustellen, daß es bisher kein zufriedenstellendes Verfahren zur Herstellung von fluorierten Benzaldehyden aus chlorierten Benzaldehyden gibt, denn diese Reaktion kann bisher nur mit unbefriedigenden Ausbeuten durchgeführt werden und liefern die gewünschten Produkte nicht in der erforderlichen Reinheit, auch wenn dabei in verdünntem Medium oder mit zwei Katalysatoren gearbeitet wird und, daß bisher die Herstellung von fluorierten Benzylalkoholen durch Reduktion entweder aufwendig zu handhabende und teure Reduktionsmittel erfordert oder von schwer zugänglichen Ausgangsprodukten ausgeht oder, im Falle der Reduktion entsprechender Aldehyde mit Wasserstoff in Gegenwart von Raney-Nickel, nur mäßige Ausbeuten erzielt werden. Die Ausbeuten und Reinheiten der nach den bekannten Verfahren erhältlichen Produkte genügen insbesondere nicht den Anforderungen für die Synthese von Wirkstoffen.

Es wurde nun ein Verfahren zur Herstellung von fluorierten Benzylalkoholen der Formel (I) gefunden 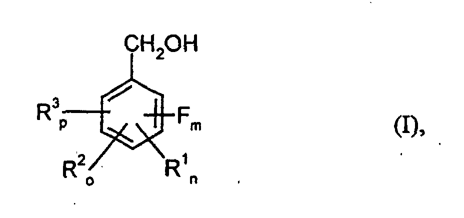 in der
- R¹: für C₁-C₄-Fluoralkyl,
- R²: für Chlor,
- R³: für C₁-C₈-Alkyl,
- m: für eine ganze Zahl von 1 bis 4 und
n, o und p unabhängig voneinander jeweils für Null oder eine ganze Zahl von 1 bis 3 stehen,
wobei gilt: m+n+o+p ≤5,
das dadurch gekennzeichnet ist, daß man chlorierte Benzaldehyde der Formel (II) 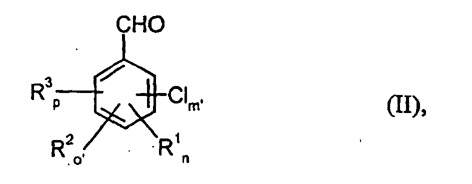 in der
- R¹, R², R³, n und p: die bei Formel (I) angegebene Bedeutung haben und
- m': für Null oder eine ganze Zahl von 1 bis 3 und
- o': für eine ganze Zahl von 1 bis 3 stehen,
wobei gilt: m'+n+o'+p ≤5,
mit Alkalimetallfluoriden in Gegenwart von weniger als 2 Mol-% quartären Phosphoniumsalzen (bezogen auf auszutauschende Chloratome) und bei Ausgangskonzentrationen von mehr als 2,5 Mol des chlorierten Benzaldehyds der Formel (II) pro kg dipolar-aprotisches Lösungsmittel bei Temperaturen im Bereich 130 bis 200°C umsetzt, so fluorierte Benzaldehyde der Formel (III) erhält 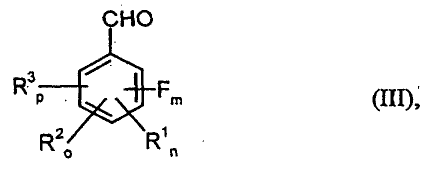 in der die verwendeten Symbole die bei Formel (I) angegebene Bedeutung haben,
und diese mit Wasserstoff in Gegenwart von Edelmetallkatalysatoren hydriert.

Es wurde auch ein Verfahren zur Herstellung von fluorierten Benzaldehyden der Formel (III) gefunden, das dadurch gekennzeichnet ist, daß man chlorierte Benzaldehyde der Formel (II) mit Alkalimetallfluoriden in Gegenwart von weniger als 2 Mol-% quartären Phosphoniumsalzen (bezogen auf auszutauschende Chloratome) und bei Ausgangskonzentrationen von mehr als 2,5 Mol des chlorierten Benzaldehyds der Formel (II) pro kg dipolar-aprotisches Lösungsmittel bei Temperaturen im Bereich 130 bis 200°C umsetzt.

Die in den Formeln (I) bis (III) verwendeten Symbole haben vorzugsweise folgende Bedeutungen:
- R¹ =: Trifluormethyl oder Pentafluorethyl.
- R² =: Chlor.
- R³ =: C₁-C₄-Alkyl, insbesondere Methyl und Ethyl.
- m =: eine ganze Zahl von 1 bis 3, insbesondere 1 oder 2.
n, o und p unabhängig voneinander Null oder 1,
wobei gilt m+n+o+p ≤3.
- m' =: Null, 1 oder 2 und
- o' =: 1 oder 2,
wobei gilt m'+n+o'+p ≤3.

Besonders bevorzugt wird in die erfindungsgemäßen Verfahren 2,6-Dichlorbenzaldehyd eingesetzt und 2-Fluor-6-chlorbenzaldehyd, 2-Fluor-6-chlorbenzylalkohol, 2,6-Difluorbenzaldehyd oder 2,6-Difluorbenzylalkohol erhalten oder 2,4-Dichlorbenzaldehyd eingesetzt und 2,4-Difluorbenzaldehyd oder 2,4-Difluorbenzylalkohol erhalten oder 3,4-Dichlorbenzaldehyd eingesetzt und 3-Chlor-4-fluorbenzaldehyd oder 3-Chlor-4-fluorbenzylalkohol erhalten.

Die als Einsatzmaterial benötigten chlorierten Benzaldehyde der Formel (II) sind aus den entsprechenden Toluolen einfach und kostengünstig durch Kern- und Seitenkettenchlorierung und anschließende Hydrolyse zu erhalten.

Als Alkalimetallfluoride kommen z.B. Natrium-, Kalium-, Rubidium- und Cäsiumfluorid oder Mischungen davon in Frage. Es ist vorteilhaft, die eingesetzten Alkalimetallfluoride vor der Reaktion zu entwässern, z.B. indem man sie sprühtrocknet oder zunächst das Alkalimetallfluorid mit Lösungsmitteln erhitzt und dabei ein Gemisch aus Wasser und Lösungsmittel abdestilliert. Bevorzugt gelangt Kaliumfluorid zum Einsatz, gegebenenfalls mit Zumischungen von Cäsiumfluorid. Alkalimetallfluoride können z.B. in Mengen von 0,8 bis 2 Molen pro Mol auszutauschender Chloratome eingesetzt werden. Vorzugsweise beträgt diese Menge 1,1 bis 1,6 Mol.

Folgende weitere Maßnahmen sind unabhängig voneinander bei der erfindungsgemäßen Fluorierung bevorzugt:
- Gegenwart von weniger als 1,8 Mol-% quartärer Phosphoniumsalze (bezogen auf auszutauschende Chloratome).
- Verwendung von Tetraorganophosphoniumhalogeniden, insbesondere Tetraphenylphosphoniumbromid als quartäres Phosphoniumsalz.
- Ausgangskonzentration von mehr als 2,8 Mol des chlorierten Aldehyds der Formel (II) pro kg dipolar-aprotisches Lösungsmittel.
- Einsatz von Diphenylsulfon, Tetramethylensulfon, Dimethylsulfoxid, Tetramethylensulfoxid, Dimethylacetamid, Dimethylformamid, N-Methylpyrrolidon oder beliebigen Mischungen davon, insbesondere von Tetramethylensulfon, als dipolar-aprotisches Lösungsmittel.
- Reaktionstemperaturen im Bereich 150 bis 200°C.
- Einsatz möglichst wasserarmer chlorierter Aldehyde der Formel (II) und möglichst wasserarmer dipolar-aprotischer Lösungsmittel.
- Durchführung der Reaktion und Handhabung der chlorierten und fluorierten Benzaldehyde unter Schutzgasatmosphäre.

Nach Beendigung der Fluorierungsreaktion kann man den hergestellten fluorierten Benzaldehyd der Formel (III) aus dem Reaktionsgemisch beispielsweise durch Herausdestillieren unter vermindertem Druck abtrennen. Gegebenenfalls kann man ihn weiter reinigen, z.B. durch fraktionierte Destillation unter vermindertem Druck. Man erhält die fluorierten Benzaldehyde der Formel (III) als isoliertes Produkt i.a. in Ausbeuten von über 75 %.

Es ist vorteilhaft, den entstehenden fluorierten Benzaldehyd der Formel (III) kontinuierlich aus dem Reaktionsgemisch abzudestillieren und/oder das Edukt dem Reaktionsgemisch kontinuierlich zuzuführen.

Für die erfindungsgemäße katalytische Hydrierung kommen vorzugsweise Palladium-, Platin- und Rutheniumkatalysatoren, insbesondere Palladiumkatalysatoren, in Frage.

Vorzugsweise ist das Edelmetall auf einem Trägermaterial aufgebracht. Trägermaterialien können z.B. Kieselsäuren, Aluminiumoxide, Silikate, Carbonate, Sulfate oder Kohlen der verschiedensten Art sein. Bevorzugte Trägermaterialien sind Kohlen. Besonders bevorzugte Edelmetallkatalysatoren sind Palladium-auf-Kohle-Katalysatoren. Die Trägerkatalysatoren können z.B. 0,1 bis 20 Gew.-% Edelmetall, bezogen auf das Gewicht des Trägermaterials, enthalten. Vorzugsweise beträgt diese Menge 0,5 bis 15 Gew.-%, insbesondere 1 bis 10 Gew.-%. Geeignete Edelmetallkatalysatoren, insbesondere solche, die Palladium-auf-Kohle enthalten, sind im Handel erhältlich.

Die Edelmetallkatalysatoren, insbesondere Edelmetallträgerkatalysatoren (dann nur deren Edelmetallanteil berücksichtigt), können z.B. in Mengen von 0,00001 bis 5 Gew.-%, bezogen auf den fluorierten Aldehyd der Formel (III) eingesetzt werden. Bevorzugt beträgt diese Menge 0,0001 bis 2 Gew.-%.

Die erfindungsgemäße katalytische Hydrierung kann mit oder ohne Lösungsmittel durchgeführt werden. Geeignete Lösungsmittel sind z.B. unter Reaktionsbedingungen flüssige aliphatische und aromatische Kohlenwasserstoffe, Ether, Ester und Alkohole, insbesondere C₅-C₁₂-Aliphaten, C₆-C₁₀-Aromaten, C₇-C₁₀-Alkylaromaten, Di-C₂-C₅-alkylether (auch unsymmetrische), cyclische Ether, Ester mit insgesamt 3 bis 15 C-Atomen und Alkohole mit 1 bis 12 C-Atomen. Ohne Lösungsmittel kann man dann arbeiten, wenn der eingesetzte fluorierte Aldehyd der Formel (III) unter Reaktionsbedingungen flüssig ist. Vorzugsweise arbeitet man in Gegenwart von C₁-C₄-Alkylalkoholen oder Toluol oder ohne Lösungsmittel-Zusätze.

Wenn man Lösungsmittel einsetzt ist die Lösungsmittelmenge nicht kritisch. Beispielsweise kann man, bezogen auf fluorierten Benzaldehyd der Formel (III), 0 bis 1 500 Gew.-% Lösungsmittel verwenden.

Die erfindungsgemäße Hydrierung kann z.B. bei Temperaturen im Bereich -30 bis +300°C durchgeführt werden. Bevorzugt liegt die Temperatur im Bereich 0 bis 150°C, insbesondere im Bereich 20 bis 100°C.

Die erfindungsgemäße Hydrierung kann z.B. bei Drücken im Bereich 1 bis 300 bar durchgeführt werden. Bevorzugt liegt der Druck im Bereich von 2 bis 150 bar, insbesondere im Bereich von 5 bis 100 bar.

Das nach Beendigung der Hydrierung vorliegende Raektionsgemisch kann man beispielsweise aufarbeiten, indem man die festen Bestandteile abtrennt, z.B. durch Filtration, und aus dem Filtrat gegebenenfalls vorhandenes Lösungsmittel abdestilliert. Man erhält die fluorierten Benzylalkohole der Formel (I) nach der Durchführung der Hydrierung i.a. in Ausbeuten von 80 % und höher, häufig von 90 % und höher.

Man kann die katalytische Hydrierung z.B. als Sumpfphasen-, Rieselphasen- oder Gasphasenhydrierung an fest angeordneten Edelmetallkatalysatoren durchführen.

Die erfindungsgemäßen Verfahren gestatten die Herstellung von fluorierten Benzylaldehyden und -alkoholen in Ausbeuten, die deutlich höher liegen als beim Stand der Technik. Außerdem werden reinere Produkte erhalten als bisher.

Darüber hinaus wird für die Fluorierung nur ein Katalysatortyp in geringer Menge benötigt, und es kann in konzentrierter Lösung gearbeitet werden. Bei der Hydrierung werden schwierig zu handhabende und teure Reduktionsmittel vermieden und trotzdem um über 10 % höhere Ausbeuten erzielt.

### Beispiele

### Beispiel 1

Unter Stickstoff wurde eine Suspension von 348 g Kaliumfluorid in 700 ml Tetramethylensulfon 1,5 Stunden lang bei 100°C gerührt. Anschließend wurden 200 ml des Flüssigkeitsvolumens abdestilliert. Im Stickstoffgegenstrom erfolgte nun die Zugabe von 350 g 2,6-Dichlorbenzaldehyd. Die Reaktionsmischung wurde nun nach Zugabe von 20,4 g Tetraphenylphosphoniumbromid unter Stickstoff und Rühren für 15 Stunden auf 170°C erhitzt. Der Verlauf der Reaktion wurde gaschromatografisch verfolgt. Nach Beendigung der Reaktion wurde 2,6-Difluorbenzaldehyd aus der Reaktionsmischung herausdestilliert (Siedebereich bis 145°C bei 18 mbar) und im Anschluß durch fraktionierte Destillation von Verunreinigungen befreit. Der Siedepunkt des 2,6-Difluorbenzaldehyds betrug dabei 68 bis 70°C bei 11 mbar. Die Ausbeute betrug 80 % der Theorie, die Reinheit des Produktes lag bei über 98 % (GC).

### Beispiel 2

Unter Stickstoff wurde eine Suspension von 348 g Kaliumfluorid in 700 ml Tetramethylensulfon 1,5 Stunden bei 100°C gerührt. Anschließend wurden 200 ml des Flüssigkeitsvolumens abdestilliert. Im Stickstoffgegenstrom erfolgte nun die Zugabe von 350 g 2,4-Dichlorbenzaldehyd. Die Reaktionsmischung wurde nun nach Zugabe von 30 g Tetraphenylphosphoniumbromid unter Stickstoff und Rühren für 16 Stunden auf 160°C erhitzt. Der Verlauf der Reaktion wurde gaschromatografisch verfolgt. Nach der Beendigung der Reaktion wurde 2,4-Difluorbenzaldehyd aus der Reaktionsmischung herausdestilliert (Siedebereich bis 145°C bei 18 mbar) und im Anschluß daran durch fraktionierte Destillation von Verunreinigungen befreit. Der Siedepunkt des 2,4-Difluorbenzaldehyds betrug dabei 48°C bei 11 mbar. Die Ausbeute lag bei 79 % der Theorie, die Reinheit bei über 98 % (GC).

### Beispiele 3 bis 8

Der jeweils eingesetzte 2,6-Difluorbenzaldehyd war entsprechend Beispiel 1 erhalten worden.

### Beispiel 3

332 g 2,6-Difluorbenzaldehyd wurden in einem 0,7 l Autoklaven unter Stickstoff vorgelegt und mit 6,4 g 5 gew.%igem Palladium-auf-Kohle (Katalysator der Fa.Heraeus Typ K-0227, enthaltend 50 Gew.-% Wasser) versetzt. Der Autoklav wurde verschlossen und mit Stickstoff gespült. Danach wurden 50 bar Wasserstoff aufgedrückt und auf 80°C erwärmt. Es wurde insgesamt 4 Stunden unter Rühren hydriert. Nach dem Abkühlen und Entspannen wurde das Reaktionsgemisch über Celite®-Filtrierhilfe filtriert, der Autoklav mit 50 g Isopropanol ausgespült und der Filterkuchen mit 150 g Isopropanol gewaschen. Aus den vereinigten Filtraten und Waschwässern wurde das Lösungsmittel abdestilliert und das rohe Produktgemisch anschließend destilliert. Es wurden 288 g 2,6-Difluorbenzylalkohol erhalten (90 % der Theorie).

### Beispiel 4

5 g 2,6-Difluorbenzaldehyd wurden in 100 ml Isopropanol gelöst, mit 1 g des auch in Beispiel 3 verwendeten Katalysators versetzt und das Gemisch in einen Autoklaven überführt. Danach wurden 50 bar Wasserstoff aufgedrückt und auf 50°C erwrärmt. Es wurde insgesamt 5 Stunden unter Rühren hydriert. Danach wurde das Reaktionsgemisch filtriert und aus dem Filtrat Isopropanol abdestilliert. Die Rohausbeute an 2,6-Difluorbenzylalkohol betrug 94 % der Theorie.

### Beispiel 5

5 g 2,6-Difluorbenzaldehyd wurden in 100 ml Isopropanol gelöst, mit 0,075 g des auch in Beispiel 3 verwendeten Katalysators versetzt und in einen Autoklaven überführt. Danach wurden 50 bar Wasserstoff aufgedrückt und auf 80°C erwärmt. Es wurde insgesamt 5 Stunden unter Rühren hydriert. Danach wurde das Reaktionsgemisch abfiltriert und aus dem Filtrat Isopropanol abdestilliert. Die Rohausbeute an 2,6-Difluorbenzylalkohol betrug 95 % der Theorie.

### Beispiel 6

7,5 g 2,6-Difluorbenzaldehyd wurden in 100 ml Isopropanol gelöst, mit 1,25 g 1 gew.-%igem Platin-auf-Kohle (feucht, 50 Gew.-% Wasser) als Katalysator versetzt und das Gemisch in einen Autoklaven überführt. Danach wurden 50 bar Wasserstoff aufgedrückt und auf 50°C erwärmt. Es wurde insgesamt 10 Stunden unter Rühren hydriert. Danach wurde das Reaktionsgemisch filtriert und aus dem Filtrat Isopropanol abdestilliert. Die Rohausbeute an 2,6-Difluorbenzylalkohol betrug 92 % der Theorie.

### Beispiel 7

7,5 g 2,6-Difluorbenzaldehyd wurden in 100 ml Isopropanol gelöst, mit 1,25 g des auch in Beispiel 6 verwendeten Katalysators versetzt und das Gemisch in einen Autoklaven überführt. Danach wurden 50 bar Wasserstoff aufgedrückt und auf 80°C erwärmt. Es wurde insgesamt 10 Stunden unter Rühren hydriert. Danach wurde die Reaktionslösung filtriert und aus dem Filtrat Isopropanol abdestilliert. Die Rohausbeute an 2,6-Difluorbenzylalkohol betrug 84 % der Theorie.

### Beispiel 8

5g 2,6-Difluorbenzaldehyd wurden in 100 ml Isopropanol gelöst, mit 1,5 g 5 gew.-%igem Ruthenium-auf-Kohle (feucht, 50 Gew.-% Wasser) als Katalysator versetzt und das Gemisch in einen Autoklaven gegeben. Danach wurden 50 bar Wasserstoff aufgedrückt und auf 50°C erwärmt. Es wurde insgesamt 5 Stunden unter Rühren hydriert. Danach wurde die Reaktionslösung abfiltriert und aus dem Filtrat Isopropanol abdestilliert. Die Rohausbeute an 2,6-Difluorbenzylalkohol betrug 80 % der Theorie.

### Beispiel 9

2,5 g 2,4-Difluorbenzaldehyd (erhalten gemäß Beispiel 2) wurden in 20 ml Toluol gelöst, mit 0,2 g des auch in Beispiel 3 verwendeten Katalysators versetzt und das Gemisch in einen Autoklaven gegeben. Danach wurden 50 bar Wasserstoff aufgedrückt und für 10 Stunden auf 80°C erwärmt. Danach wurde das Reaktionsgemisch filtriert und aus dem Filtrat Toluol abdestilliert. Die Ausbeute an 2,4-Difluorbenzylalkohol betrug 98 % der Theorie.

### Beispiel 10 (Vergleichsbeispiel zur DE-A 36 37 156)

a) Unter Stickstoff wurde eine Suspension von 348 g Kaliumfluorid in 700 ml Tetramethylensulfon 1,5 Stunden bei 100°C gerührt. Anschließend wurden 200 ml des Flüssigkeitsvolumen abdestilliert. Im Stickstoffgegenstrom erfolgte nun die Zugabe von 350 g 2,6-Dichlorbenzaldehyd. Die Reaktionsmischung wurde unter Stickstoff und Rühren für 33 Stunden auf 170°C erhitzt. Nach Beendigung der Reaktion wurden die Produkte aus der Reaktionsmischung herausdestilliert (Siedebereich: bis 145°C bei 18 mbar) und im Anschluß durch fraktionierte Destillation vom Lösungsmittel und Verunreinigungen befreit (Kp.₁₁: 68-70°C).

| | | |
|---|---|---|
| Ausbeute | 65 g = 23 % d.Th. | 2-Chlor-6-fluorbenzaldehyd |
| | nur Spuren | 2,6-Difluorbenzaldehyd |

b) Experiment a) wurde mit einer Reaktionstemperatur von 220°C und einer Reaktionszeit von 20 Stunden wiederholt. Dabei wurde 2,6-Difluorbenzaldehyd in einer Ausbeute von 44 % d.Th. neben 22 % d.Th. 2-Chlor-6-fluorbenzaldehyd erhalten. Das Edukt war nur noch in Spuren nachzuweisen.

## Patentansprüche

1. Verfahren zur Herstellung von fluorierten Benzylalkoholen der Formel (I) 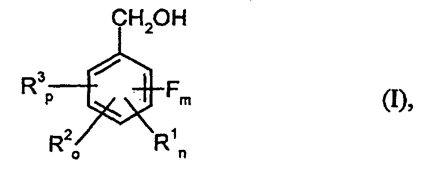 in der
R¹ für C₁-C₄-Fluoralkyl,
R² für Chlor,
R³ für C₁-C₈-Alkyl,
m für eine ganze Zahl von 1 bis 4 und
n, o und p unabhängig voneinander jeweils für Null oder eine ganze Zahl von 1 bis 3 stehen,
wobei gilt: m+n+o+p ≤5,
**dadurch gekennzeichnet, daß** man chlorierte Benzaldehyde der Formel (II) 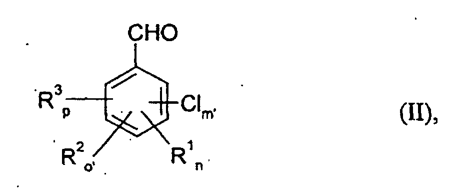 in der
R¹, R², R³, n und p die bei Formel (I) angegebene Bedeutung haben und
m' für Null oder eine ganze Zahl von 1 bis 3 und
o' für eine ganze Zahl von 1 bis 3 stehen,
wobei gilt: m'+n+o'+p ≤5,
mit Alkalimetallfluoriden in Gegenwart von weniger als 2 Mol-% quartären Phosphoniumsalzen (bezogen auf auszutauschende Chloratome) und bei Ausgangskonzentrationen von mehr als 2,5 Mol des chlorierten Benzaldehyds der Formel (II) pro kg dipolar-aprotisches Lösungsmittel bei Temperaturen im Bereich 130 bis 200°C umsetzt, so fluorierte Benzaldehyde der Formel (III) erhält 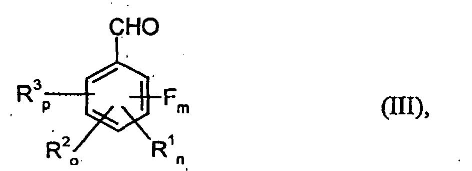 in der die verwendeten Symbole die bei Formel (I) angegebene Bedeutung haben,
und diese mit Wasserstoff in Gegenwart von Edelmetallkatalysatoren hydriert.

2. Verfahren zur Herstellung von fluorierten Benzaldehyden der Formel (III) 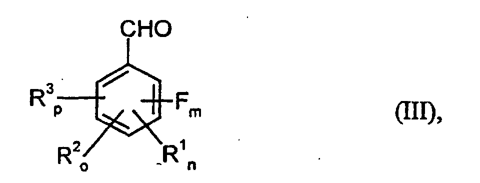 in der
R¹ für C₁-C₄-Fluoralkyl,
R² für Chlor,
R³ für C₁-C₈-Alkyl,
m für eine ganze Zahl von 1 bis 4 und
n, o und p unabhängig voneinander jeweils für Null oder eine ganze Zahl von 1 bis 3 stehen,
wobei gilt: m+n+o+p ≤5,
**dadurch gekennzeichnet, daß** man chlorierte Benzaldehyde der Formel (II) 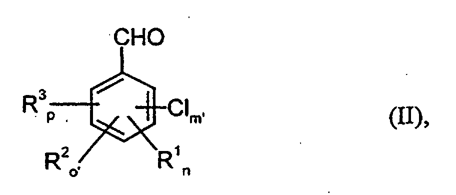 in der
R¹, R², R³, n und p die bei Formel (I) angegebene Bedeutung haben und
m' für Null oder eine ganze Zahl von 1 bis 3 und
o' für eine ganze Zahl von 1 bis 3 stehen,
wobei gilt: m'+n+o'+p ≤5,
mit Alkalimetallfluoriden in Gegenwart von weniger als 2 Mol-% quartären Phosphoniumsalzen (bezogen auf auszutauschende Chloratome) und bei Ausgangskonzentrationen von mehr als 2,5 Mol des chlorierten Benzaldehyds der Formel (II) pro kg dipolar-aprotisches Lösungsmittel bei Temperaturen im Bereich 130 bis 200°C umsetzt.

3. Verfahren nach Ansprüchen 1 und 2, **dadurch gekennzeichnet, daß** in den Formeln (I) bis (III) die verwendeten Symbole folgende Bedeutungen haben:
R¹ = Trifluormethyl oder Pentafluorethyl,
R² = Chlor,
R³ = C₁-C₄-Alkyl,
m = eine ganze Zahl von 1 bis 3,
n, o und p unabhängig voneinander Null oder 1, wobei gilt m+n+o+p ≤3,
m' = Null, 1 oder 2 und
o' = 1 oder 2, wobei gilt m'+n+o'+p ≤3.

4. Verfahren nach Ansprüchen 1 bis 3, **dadurch gekennzeichnet, daß** man 2,6-Dichlorbenzaldehyd einsetzt und 2-Fluor-6-chlorbenzaldehyd, 2-Fluor-6-chlorbenzylalkohol, 2,6-Difluorbenzaldehyd oder 2,6-Difluorbenzylalkohol erhält oder 2,4-Dichlorbenzaldehyd einsetzt und 2,4-Difluorbenzaldehyd oder 2,4-Difluorbenzylalkohol erhält oder 3,4-Dichlorbenzaldehyd einsetzt und 3-Chlor-4-fluorbenzaldehyd oder 3-Chlor-4-fluorbenzylalkohol erhält.

5. Verfahren nach Ansprüchen 1 bis 4, **dadurch gekennzeichnet, daß** man als Alkalimetallfluoride Kaliumfluorid, gegebenenfalls mit Zumischungen von Cäsiumfluorid einsetzt.

6. Verfahren nach Ansprüchen 1 bis 5, **dadurch gekennzeichnet, daß** man es in Gegenwart von weniger als 1,8 Mol-% quartärer Phosphoniumsalze (bezogen auf auszutauschende Chloratome) durchführt, es mit einer Ausgangskonzentration von mehr als 2,8 Mol des chlorierten Aldehyds der Formel (II) pro kg dipolar-aprotisches Lösungsmittel beginnt, Tetramethylensulfon als dipolar-aprotisches Lösungsmittel einsetzt, Reaktionstemperaturen im Bereich von 150 bis 200°C einhält und die Durchführung der Reaktion und Handhabung der chlorierten und fluorierten Benzaldehyde unter Schutzgasatmosphäre vornimmt.

7. Verfahren nach Anspruch 1, **dadurch gekennzeichnet, daß** man die katalytische Hydrierung mit Palladium-, Platin- und/oder Rutheniumkatalysatoren vornimmt, die auf Trägermaterial aufgebracht sind.

8. Verfahren nach Ansprüchen 1 und 7, **dadurch gekennzeichnet, daß** die Trägerkatalysatoren 0,1 bis 20 Gew.-% Edelmetall, bezogen auf das Trägermaterial, enthalten.

9. Verfahren nach Ansprüchen 1, 7 und 8, **dadurch gekennzeichnet, daß** man die katalytische Hydrierung in Gegenwart von aliphatischen oder aromatischen Kohlenwasserstoffen, Ethern, Estern und/oder unter Reaktionsbedingungen flüssigen Alkoholen durchführt.

10. Verfahren nach Ansprüchen 1 bis 9, **dadurch gekennzeichnet, daß** man den entstehenden fluorierten Benzaldehyd der Formel (III) kontinuierlich aus dem Reaktionsgemisch abdestilliert und/oder das Edukt dem Reaktionsgemisch kontinuierlich zuführt.

## Claims

1. Process for preparing fluorinated benzyl alcohols of the formula (I) 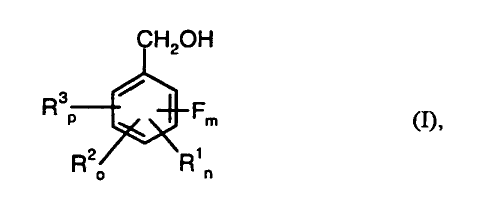 where
R¹ represents C₁-C₄-fluoroalkyl,
R² represents chlorine,
R³ represents C₁-C₈-alkyl,
m represents an integer from 1 to 4 and
n, o and p each represent, independently of one another, zero or an integer from 1 to 3,
where: m+n+o+p ≤5,
**characterized in that** chlorinated benzaldehydes of the formula (II) 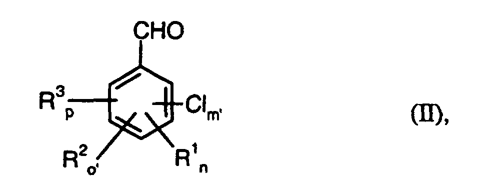 where
R¹, R², R³, n and p are as defined for formula (I) and
m' represents zero or an integer from 1 to 3 and
o' represents an integer from 1 to 3,
where: m'+n+o'+p ≤5,
are reacted with alkali metal fluorides at temperatures in the range from 130 to 200°C in the presence of less than 2 mol% of quaternary phosphonium salts (based on chlorine atoms to be replaced) at initial concentrations of more than 2.5 mol of the chlorinated benzaldehyde of the formula (II) per kg of dipolar aprotic solvent to give fluorinated benzaldehydes of the formula (III) 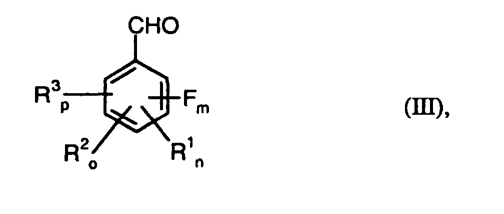 where the symbols used are as defined for formula (I), and these are hydrogenated with hydrogen in the presence of noble metal catalysts.

2. Process for preparing fluorinated benzaldehydes of the formula (III) 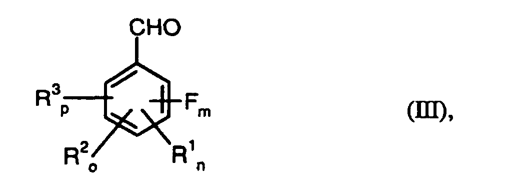 where
R¹ represents C₁-C₄-fluoroalkyl,
R² represents chlorine,
R³ represents C₁-C₈-alkyl,
m represents an integer from 1 to 4 and
n, o and p each represent, independently of one another, zero or an integer from 1 to 3,
where: m+n+o+p ≤5,
**characterized in that** chlorinated benzaldehydes of the formula (II) 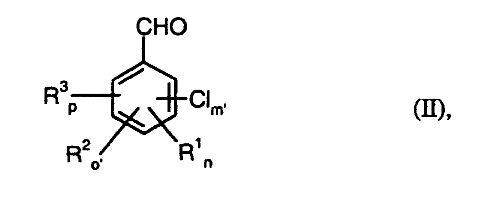 where
R¹, R², R³, n and p are as defined for formula (I) and
m' represents zero or an integer from 1 to 3 and
o' represents an integer from 1 to 3,
where: m'+n+o'+p ≤5,
are reacted with alkali metal fluorides at temperatures in the range from 130 to 200°C in the presence of less than 2 mol% of quaternary phosphonium salts (based on chlorine atoms to be replaced) at initial concentrations of more than 2.5 mol of the chlorinated benzaldehyde of the formula (II) per kg of dipolar aprotic solvent.

3. Process according to Claim 1 or 2, **characterized in that** the symbols used in the formulae (I) to (III) have the following meanings:
R¹ = trifluoromethyl or pentafluoroethyl,
R² = chlorine,
R³ = C₁-C₄-alkyl,
m = an integer from 1 to 3,
n, o and p, independently of one another = zero or 1, where m+n+o+p ≤3,
m' = zero, 1 or 2 and
o' = 1 or 2, where m'+n+o'+p ≤3.

4. Process according to any of Claims 1 to 3, **characterized in that** 2,6-dichlorobenzaldehyde is used and 2-fluoro-6-chlorobenzaldehyde, 2-fluoro-6-chlorobenzyl alcohol, 2,6-difluorobenzaldehyde or 2,6-difluorobenzyl alcohol is obtained or 2,4-dichlorobenzaldehyde is used and 2,4-difluorobenzaldehyde or 2,4-difluorobenzyl alcohol is obtained or 3,4-dichlorobenzaldehyde is used and 3-chloro-4-fluorobenzaldehyde or 3-chloro-4-fluorobenzyl alcohol is obtained.

5. Process according to any of Claims 1 to 4, **characterized in that** the alkali metal fluoride is potassium fluoride, if desired in admixture with caesium fluoride.

6. Process according to any of Claims 1 to 5, **characterized in that** it is carried out in the presence of less than 1.8 mol% of quaternary phosphonium salts (based on chlorine atoms to be replaced), it is commenced at an initial concentration of more than 2.8 mol of the chlorinated aldehyde of the formula (II) per kg of dipolar aprotic solvent, tetramethylene sulphone is used as dipolar aprotic solvent, reaction temperatures in the range from 150 to 200°C are maintained and the reaction procedure and the handling of the chlorinated and fluorinated benzaldehydes are carried out under a protective gas atmosphere.

7. Process according to Claim 1, **characterized in that** the catalytic hydrogenation is carried out using palladium, platinum and/or ruthenium catalysts which are applied to support materials.

8. Process according to Claim 1 or 7, **characterized in that** the supported catalysts contain from 0.1 to 20% by weight of noble metal, based on the support material.

9. Process according to Claim 1, 7 or 8, **characterized in that** the catalytic hydrogenation is carried out in the presence of aliphatic or aromatic hydrocarbons, ethers, esters and/or alcohols which are liquid under the reaction conditions.

10. Process according to any of Claims 1 to 9, **characterized in that** the fluorinated benzaldehyde of the formula (III) which is being formed is continuously distilled from the reaction mixture and/or the starting material is continuously fed to the reaction mixture.

## Revendications

1. Procédé pour la préparation d'alcools benzyliques fluorés de formule (I) 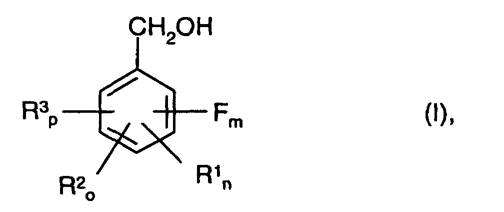 dans laquelle
R¹ représente un groupe fluoralkyle en C₁-C₄,
R² représente le chlore,
R³ représente un groupe alkyle en C₁-C₈,
m est un nombre entier allant de 1 à 4 et
n, o et p sont égaux chacun, indépendamment les uns des autres, à zéro ou à un nombre entier allant de 1 à 3,
et m+n+o+p ≤ 5,
**caractérisé en ce que** l'on fait réagir des benzaldéhydes chlorés de formule (II) 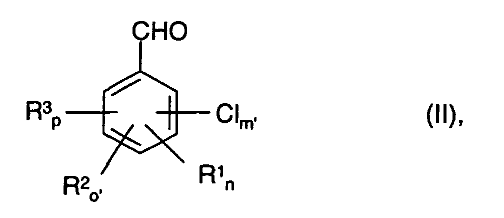 dans laquelle
R¹, R², R³, n et p ont les significations indiquées en référence à la formule (I) et
m' est égal à zéro ou à un nombre entier allant de 1 à 3, et
o' est un nombre entier allant de 1 à 3,
et
m'+n+o'+p ≤ 5,
Le A 33 066 (0 979 812)
avec des fluorures de métaux alcalins en présence de moins de 2 mol % de sels de phosphonium quaternaire (par rapport aux atomes de chlore à échanger) et à des concentrations initiales supérieures à 2,5 mol du benzaldéhyde chloré de formule (II) par kilogramme de solvant aprotonique dipolaire, à des températures dans l'intervalle de 130 à 200°C, ce qui donne des benzaldéhydes fluorés de formule (III) 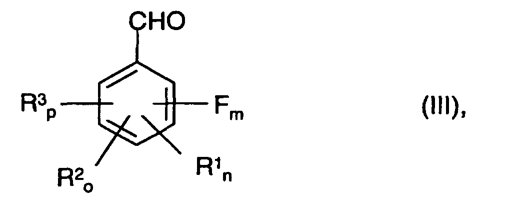 dans laquelle les symboles ont les significations indiquées en référence à la formule (I),
qu'on hydrogène en présence de catalyseurs à base de métaux nobles.

2. Procédé pour la préparation de benzaldéhydes fluorés de formule (III) 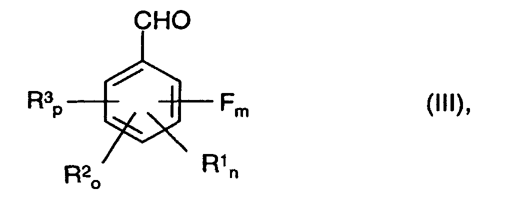 dans laquelle
R¹ représente un groupe fluoralkyle en C₁-C₄,
R² représente le chlore,
R³ représente un groupe alkyle en C₁-C₈,
m est un nombre entier allant de 1 à 4, et
n, o et p sont égaux chacun, indépendamment les uns des autres, à zéro ou à un nombre entier allant de 1 à 3,
et
m+n+o+p ≤ 5,
**caractérisé en ce que** l'on fait réagir des benzaldéhydes chlorés de formule (II) 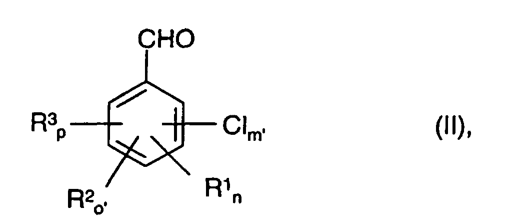 dans laquelle
R¹, R², R³, n et p ont les significations indiquées en référence à la formule (I) et
m' est égal à zéro ou à un nombre entier allant de 1 à 3 et
o' est un nombre entier allant de 1 à 3,
et
m'+n+o'+p ≤ 5,
avec des fluorures de métaux alcalins en présence de moins de 2 mol % de sels de phosphonium quaternaire (par rapport aux atomes de chlore à échanger) et à des concentrations initiales supérieures à 2,5 mol du benzaldéhyde chloré de formule (II) par kilogramme de solvant aprotonique dipolaire, à des températures dans l'intervalle de 130 à 200°C.

3. Procédé selon les revendications 1 et 2, **caractérisé en ce que** les symboles des formules (I) à (III) ont les significations suivantes :
R¹ = trifluorométhyle ou pentafluoréthyle,
R² = chlore,
R³ = alkyle en C₁-C₄,
m = nombre entier de 1 à 3,
n, o et p, indépendamment les uns des autres, 0 ou 1, et m+n+o+p ≤ 3,
m' = 0, 1 ou 2 et
o' = 1 ou 2,
et
m'+n+o'+p ≤ 3.

4. Procédé selon les revendications 1 à 3, **caractérisé en ce que** l'on met en oeuvre le 2,6-dichlorobenzaldéhyde et on obtient le 2-fluoro-6-chlorobenzaldéhyde, l'alcool 2-fluoro-6-chlorobenzylique, le 2,6-difluorobenzaldéhyde ou l'alcool 2,6-difluorobenzylique ou bien on met en oeuvre le 2,4-dichlorobenzaldéhyde et on obtient le 2,4-diflurobenzaldéhyde ou l'alcool 2,4-difluorobenzylique ou bien on met en oeuvre le 3,4-dichlorobenzaldéhyde et on obtient le 3-chloro-4-fluorobenzaldéhyde ou l'alcool 3-chloro-4-benzylique.

5. Procédé selon les revendications 1 à 4, **caractérisé en ce que** le fluorure de métal alcalin mis en oeuvre est le fluorure de potassium éventuellement en mélange avec du fluorure de caesium.

6. Procédé selon les revendications 1 à 5, **caractérisé en ce que** l'on opère en présence de moins de 1,8 mol % de sels de phosphonium quaternaire (par rapport aux atomes de chlore à échanger), on commence avec une concentration initiale supérieure à 2,8 mol de l'aldéhyde chloré de formule (II) par kilogramme de solvant aprotonique dipolaire, on utilise la tétraméthylènesulfone en tant que solvant aprotonique dipolaire, on maintient des températures de réaction dans l'intervalle de 150 à 200°C et on procède à la réaction et aux manipulations des benzaldéhydes chlorés et fluorés en atmosphère de gaz protecteur.

7. Procédé selon la revendication 1, **caractérisé en ce que** l'on réalise l'hydrogénation catalytique sur des catalyseurs au palladium, au platine et/ou au ruthénium appliqués sur des matières de support.

8. Procédé selon les revendications 1 à 7, **caractérisé en ce que** les catalyseurs sur support contiennent de 0,1 à 20 % en poids de métal noble appliqué sur la matière de support.

9. Procédé selon les revendications 1, 7 et 8, **caractérisé en ce que** l'hydrogénation catalytique est réalisée en présence d'hydrocarbures, éthers, esters aliphatiques ou aromatiques et/ou dans des alcools liquides dans les conditions de réaction.

10. Procédé selon les revendications 1 à 9, **caractérisé en ce que** l'on distille le benzaldéhyde fluoré de formule (III) formé en continu du mélange de réaction et/ou on envoie le composant de départ en continu au mélange de réaction.
